# EUROPÄISCHE PATENTANMELDUNG

(11) **EP 3 456 280 A1**
(43) Veröffentlichungstag der Anmeldung: **20.03.2019**
(21) Anmeldenummer: 17191039.1
(22) Anmeldetag: 14.09.2017
(51) Int. Cl.: A61B 34/30, A61B 17/17, A61B 90/00

(54) **MEDIZINISCH-ROBOTISCHES GERÄT UND REGELUNGSVERFAHREN**

(71) Anmelder: Siemens Healthcare GmbH, 91052 Erlangen (DE)
(72) Erfinder: Mönnich, Holger, 86316 Friedberg (DE); Müller, Gunter, 90562 Heroldsberg (DE)

(57) **Zusammenfassung**

Das erfindungsgemäße medizinisch-robotische Gerät weist eine kinematischen Kette von bewegbaren Komponenten, einen am Ende der kinematischen Kette angeordneten Endeffektor in Form einer Bohrhülse, welche Bohrhülse dazu ausgebildet ist, zum Einführen einer Schraube in ein Objekt (z.B. ein Knochen eines Patienten) auf das Objekt aufgelegt zu werden, wobei die Bohrhülse an ihrem auf das Objekt auflegbaren Ende eine strukturierte Oberfläche aufweist, zumindest einen Sensor an der kinematischen Kette zum Erfassen von eine auf die Bohrhülse einwirkende Kraft repräsentierenden Werten und eine Steuerungs- und Regelungseinrichtung zur Steuerung und Regelung der kinematischen Kette auf, wobei die Steuerungs- und Regelungseinrichtung dazu ausgebildet ist, mittels Impedanzregelung unter Berücksichtigung der erfassten Werte eine ausgewählte Anpresskraft der Bohrhülse auf das Objekt innerhalb eines Toleranzbereichs zu regeln.

## Beschreibung

Die Erfindung betrifft ein medizinisch-robotisches Gerät mit einer Bohrhülse gemäß dem Patentanspruch 1 sowie ein Verfahren zur Regelung einer ausgewählten Anpresskraft einer Bohrhülse gemäß dem Patentanspruch 6.

Im Bereich der robotisch-chirurgischen Eingriffe, welche sowohl diagnostischer als auch therapeutischer Natur sein können, gilt es, stets eine größtmögliche Genauigkeit zu erreichen. Besonders wichtig ist dies auch beim Einbringen bzw. Einbohren von Schrauben oder anderen Implantaten in Knochenstrukturen. Schrauben werden sowohl in der invasiven als auch in der minimalinvasiven Chirurgie in Knochen eingebracht, um eine Stabilisierung des Knochens zu bewirken. Häufig wird bei solchen Verfahren eine Bohrhülse verwendet, welche im Allgemeinen vom Chirurgen manuell gehalten und auf den Knochen gepresst wird. Die Bohrhülse soll dabei verhindern, dass die Schraube auf dem Knochen entlangrutscht, soll die gewünschte Orientierung für die Bohrtrajektorie konstant vorgeben und das umgebende Gewebe schützen.

Bekannt sind auch halb-automatische Verfahren, bei denen die Bohrhülsen z.B. von medizinisch-robotischen Geräten gehalten und positioniert werden, um die Bohrtrajektorie zu präzisieren, die Stabilität zu verbessern und den Workflow zu vereinfachen. Für automatische oder halb-automatische Bohrverfahren sind ebenso wie auch beim manuellen Halten der Bohrhülse Patientenbewegungen (z.B. durch Atem- oder Herzbewegungen) und das Rutschen auf dem Knochen während des Bohrens ein großes Problem. Bisherige Lösungen versuchen dies durch besonders steife und starr gehaltene robotische Geräte (z.B. Roboterarme) zu lösen oder verwenden zusätzliche invasive Fixierungen für die Bohrhülse am Knochen des Patienten. Auch können zyklischen Bewegungen des Patienten zuvor aufgezeichnet und dadurch automatisch nachgeführt werden. Aus der
DE 102015223921 A1 ist ein medizinisch-robotisches Gerät bekannt, an welchem neben der Bohrhülse eine mechanische Zusatzkomponente angeordnet ist, wodurch geräteexterne Kräfte gemessen und die Position der Bohrhülse auf der Basis der Kräfte korrigiert wird.

Es ist Aufgabe der vorliegenden Erfindung, ein medizinisch-robotisches Gerät bereitzustellen, welches ermöglicht, bei einem (halb-) automatischen Verfahren die Problematik von (Patienten-)Bewegungen zu verringern und das Rutschen der Bohrhülse auf dem Knochen des Patienten zu reduzieren; des Weiteren ist es Aufgabe der Erfindung, ein Verfahren zur Nutzung eines solchen medizinisch-robotischen Geräts bereitzustellen.

Die Aufgabe wird erfindungsgemäß gelöst durch ein medizinisch-robotisches Gerät gemäß dem Patentanspruch 1 und von einem Verfahren zur Regelung einer ausgewählten Anpresskraft einer Bohrhülse gemäß dem Patentanspruch 6. Vorteilhafte Ausgestaltungen der Erfindung sind jeweils Gegenstand der zugehörigen Unteransprüche.

Das erfindungsgemäße medizinisch-robotische Gerät weist eine kinematische Kette von bewegbaren Komponenten, einen am Ende der kinematischen Kette angeordneten Endeffektor in Form einer Bohrhülse, welche Bohrhülse dazu ausgebildet ist, zum Einführen einer Schraube in ein Objekt (z.B. ein Knochen eines Patienten) auf das Objekt aufgelegt zu werden, wobei die Bohrhülse an ihrem auf das Objekt auflegbaren Ende eine strukturierte Oberfläche aufweist, zumindest einen Sensor an der kinematischen Kette zum Erfassen von eine auf die Bohrhülse einwirkende Kraft repräsentierenden Werten und eine Steuerungs- und Regelungseinrichtung zur Steuerung und Regelung der kinematischen Kette auf, wobei die Steuerungs- und Regelungseinrichtung dazu ausgebildet ist, mittels Impedanzregelung unter Berücksichtigung der erfassten Werte eine ausgewählte Anpresskraft der Bohrhülse auf das Objekt innerhalb eines Toleranzbereichs zu regeln. Die Messung der einwirkenden Kraft und Regelung der Anpresskraft kann dabei über einen vorbestimmten Zeitraum kontinuierlich stattfinden. Die Richtung der Anpresskraft ist dabei z.B. orthogonal zur Oberfläche des Knochens oder parallel zur geplanten Bohrtrajektorie für die Schraube.

Das erfindungsgemäße medizinisch-robotische Gerät bietet für (halb-) automatische Verfahren zum Einführen einer Schraube in ein Objekt, z.B. einen Knochen, eines Patienten eine besonders flexible und schonende Möglichkeit, die Bohrhülse präzise auf dem Knochen zu stabilisieren. Durch die Regelung des Anpressdrucks innerhalb eines Toleranzbereichs wird vermieden, einen besonders starken Anpressdruck ausüben zu müssen, durch welchen eventuell Verletzungen des Patienten entstehen können. Durch die Regelung kann bei Bedarf eine nur leichte Anpresskraft ausgewählt werden, welcher gerade ausreichend ist, dass die Bohrhülse den Kontakt zum Objekt (Knochen) nicht verliert. Patientenbewegungen wie Atmung oder Herzschlag können flexibel kompensiert werden und es wird durch die strukturierte Oberfläche auch beim Bohren selbst ein Rutschen der Bohrhülse auf dem Knochen vermieden. Der Workflow für den Chirurgen ist durch die automatisierte Halterung der Bohrhülse erleichtert.

Der Anpressdruck kann zuvor individuell oder manuell ausgewählt oder z.B. automatisch als empirischer Wert eingestellt werden.

Nach einer Ausgestaltung der Erfindung ist der zumindest eine Sensor als Kraft- oder Drehmomentsensor ausgebildet. So sind derartige Kraft- oder Drehmomentsensoren bekannt und besonders gut geeignet, Kräfte und/oder Drehmomente an der kinematischen Kette von robotischen Geräten zu bestimmen. Dabei werden die einwirkenden Kräfte entweder direkt gemessen oder es werden Werte gemessen, aus welchen auf einfache Weise die einwirkende Kraft bestimmt bzw. berechnet werden kann. Dies kann z.B. durch die Steuerungs- und Regelungseinrichtung oder durch eine weitere Recheneinheit durchgeführt werden.

In vorteilhafter Weise für eine besonders flexible Kompensierung von Bewegungen des Objekts (Knochens) wird die einwirkende Kraft in allen drei Dimensionen erfasst. Dadurch können alle möglichen, auf die Bohrhülse einwirkende Kräfte berücksichtigt werden, also z.B. orthogonal, lateral und longitudinal wirkende Kräfte. Es ist besonders vorteilhaft für eine umfassende Messung der einwirkenden Kräfte, an mehreren Achsen der kinematischen Kette Kraft- oder Drehmomentsensoren anzubringen. Bekannte Leichtbauroboter sind teilweise mit an sämtlichen Achsen mit Kraft- oder Drehmomentsensoren ausgestattet.

Nach einer weiteren Ausgestaltung der Erfindung weist die Bohrhülse an ihrem auf das Objekt auflegbaren Ende eine gezahnte Oberfläche auf. Dadurch ist die Bohrhülse besonders geeignet, den Kontakt zum Objekt auch bei nur leichter Anpresskraft aufrechtzuerhalten.

Nach einer Ausgestaltung der Erfindung ist die Steuerungs- und Regelungseinrichtung dazu ausgebildet, die ausgewählte Anpresskraft der Bohrhülse auf das Objekt entsprechend einem Federungssystem zu regeln. Die Bewegungen des Objekts (Knochen) werden durch die Impedanzregelung abgefedert, so dass die Bohrhülse auch unter moderater Federkraft "weich" mit der lokalen Bewegung mitgeht. Auf diese Weise lassen sich die Federkräfte in drei Dimensionen individuell einstellen.

Die Erfindung umfasst ein Verfahren zur Regelung einer ausgewählten Anpresskraft einer Bohrhülse, welche am Ende einer kinematischen Kette von bewegbaren Komponenten eines medizinisch-robotischen Geräts angeordnet ist und dazu ausgebildet ist, zum Einführen einer Schraube in ein Objekt auf das Objekt aufgelegt zu werden und welche Bohrhülse an ihrem auf das Objekt auflegbaren Ende eine strukturierte Oberfläche aufweist, auf das Objekt durch eine dem medizinisch-robotischen Gerät zugeordneten Steuerungs- und Regelungseinrichtung mit den folgenden Schritten: Aufbringen der strukturierten Oberfläche der Bohrhülse auf das Objekt (wobei hier die Bohrhülse durch ihre Strukturierung mit dem Objekt gegen Verrutschen verbunden ist, aber zunächst noch keine Anpresskraft ausgeübt wird), Ausüben einer ausgewählten Anpresskraft der Bohrhülse auf das Objekt durch das medizinisch-robotische Gerät, Erfassen einer auf die Bohrhülse einwirkenden Kraft, insbesondere in drei Dimensionen, wobei zumindest ein die Kraft repräsentierender Wert mittels zumindest eines an der kinematischen Kette angeordneten Sensors, insbesondere Kraft- oder Drehmomentsensors, gemessen wird, und Regeln der ausgewählten Anpresskraft der Bohrhülse auf das Objekt innerhalb eines Toleranzbereichs mittels Impedanzregelung unter Berücksichtigung der einwirkenden Kraft.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Ansicht eines erfindungsgemäßen medizinisch-robotischen Geräts; und
- FIG 2: eine Abfolge eines erfindungsgemäßen Verfahrens.

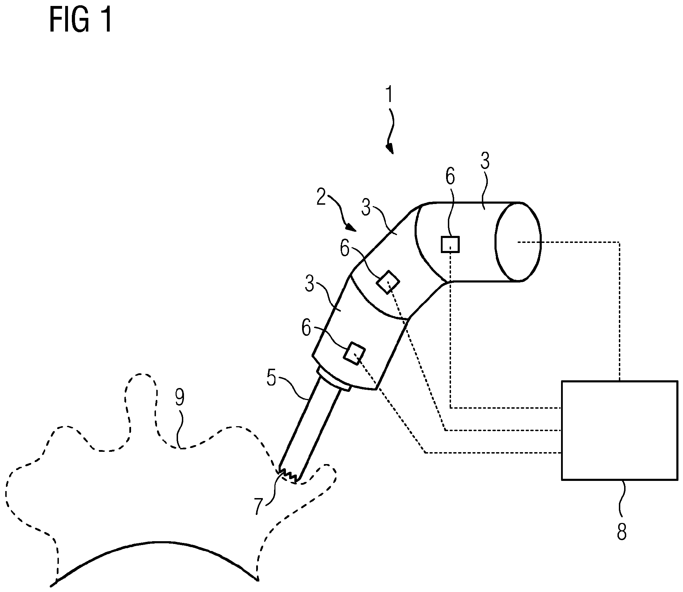

In der FIG 1 ist ein robotisches Gerät 1 mit einer kinematischen Kette 2 aus bewegbaren Komponenten 3 gezeigt. Zur Vereinfachung sind lediglich drei bewegbare Komponenten 3 gezeigt, wobei auch mehr, z.B. mindestens sechs oder sieben vorhanden sein können. Bei dem robotischen Gerät 1 kann es sich z.B. um einen Roboterarm oder Knickarmroboter oder Leichtbauroboter mit sechs- bzw. sieben Achsen handeln. Als Endeffektor ist am Ende des robotischen Geräts 1 eine Bohrhülse 5 angeordnet. Bohrhülsen 5 sind hohl geformt und werden zum Einbringen einer Schraube in ein Objekt, insbesondere einen Knochen 9 eines Patienten, mit einer bestimmten Anpresskraft auf das Objekt aufgesetzt. Die Bohrhülse 5 verhindert dabei zumindest teilweise, dass die Schraube auf dem Knochen entlangrutscht, verbessert die gewünschte Orientierung für die Bohrtrajektorie und schützt das umgebende Gewebe. Die auf den Knochen 9 des Patienten aufgesetzte Oberfläche 7 der Bohrhülse ist strukturiert ausgebildet, insbesondere ist sie gezackt geformt, so dass eine verbesserte Stabilisierung auf dem Knochen möglich ist.

An dem robotischen Gerät 1 sind als Kraftsensoren 6 ausgebildete Sensoren angeordnet, wobei im Idealfall an jeder bewegbaren Komponente 3 der kinematischen Kette 2 zumindest ein Sensor angeordnet ist. Die Kraftsensoren 6 sind dazu ausgebildet, auf die Bohrhülse einwirkende Kräfte in allen drei Dimensionen zu messen bzw. Messwerte zu messen, aus denen die einwirkende Kraft auf einfache Weise bestimmt werden kann. Es können auch Drehmomentsensoren oder andere Sensoren vorgesehen sein. Im vorliegenden Fall, dass die Bohrhülse mit einer ausgewählten Anpresskraft auf den Knochen 9 des Patienten aufgesetzt wird, handelt es sich bei der auf die Bohrhülse einwirkenden Kraft z.B. um eine durch Patientenbewegungen (z.B. Atmung, Herzschlag) ausgeübte Gegendruckkraft oder um eine Kraft, welche durch das Bohren der Schraube in den Knochen vom Chirurgen ausgeübt wird oder um eine andere geräteexterne Kraft oder um eine Summe aus mehreren derartigen Kräften. Durch die Bestimmung in allen drei Dimensionen können z.B. orthogonale, laterale oder longitudinale Kräfte gemessen werden.

Außerdem weist das robotische Gerät 1 eine Steuerungs- und Regelungseinrichtung 8 auf, welche dazu ausgebildet ist, mittels Impedanzregelung unter Berücksichtigung der einwirkenden Kraft die Anpresskraft der Bohrhülse auf das Objekt innerhalb eines Toleranzbereichs zu regeln. Die Steuerungs- und Regelungseinrichtung 8 regelt dabei mittels einer Rückkopplungsschleife die Anpresskraft der Bohrhülse auf den Knochen. Die entsprechenden einwirkenden Kräfte werden von den Kraftsensoren 6 an die Steuerungs- und Regelungseinrichtung 8 weitergegeben und von diesen für die Regelung verwendet. Es werden dabei kontinuierlich Messungen von den Kraftsensoren durchgeführt und an die Steuerungs- und Regelungseinrichtung 8 weitergegeben, welche ebenfalls kontinuierlich die Anpresskraft regelt. Insgesamt handelt es sich um eine Impedanzregelung, die eine Art Federungssystem für die Bohrhülse auf den Knochen bewirkt. Die Anpresskraft wird innerhalb eines Toleranzbereichs um den zuvor festgelegten Wert der Anpresskraft geregelt. Der Toleranzbereich bzw. die möglichen Abweichungen können zuvor eingestellt werden oder sind auf einen automatischen Standardwert festgelegt.

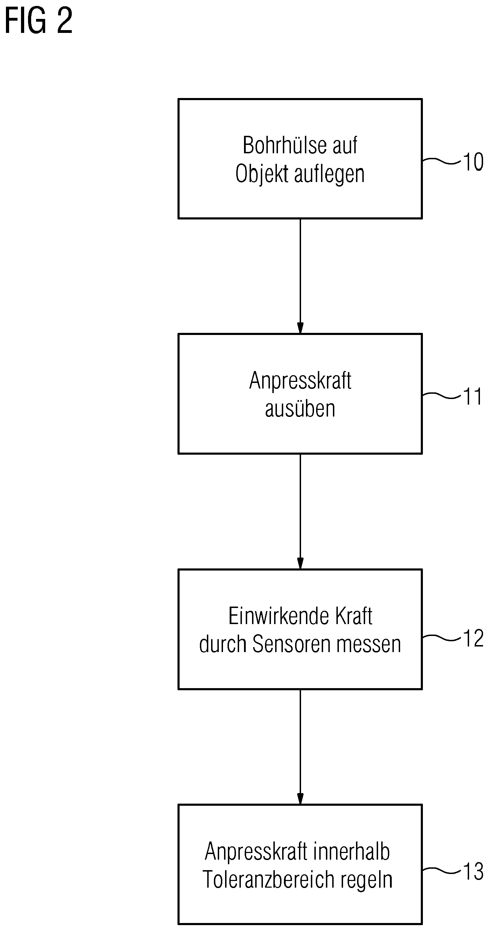

In der FIG 2 ist ein Ablauf des Verfahrens dargestellt. In einem ersten Schritt 10 wird durch die kinematische Kette 2 des robotischen Geräts 1 die Bohrhülse 5 an einem zuvor präzise festgelegten Punkt auf den Knochen 9 eines Patienten aufgelegt, wofür eine möglichst präzise Positionierung erfolgt. In diesem Schritt wird (möglichst) noch keine Anpresskraft auf das Objekt ausgeübt. Die Bohrhülse ist nach der Positionierung durch ihre Strukturierung wie z.B. ihre Zahnung mit dem Objekt gegen Verrutschen verbunden. Dies kann z.B. im Rahmen eines invasiven oder minimalinvasiven Verfahrens wie dem Einbohren einer Pedikelschraube in einen Wirbelkörper stattfinden. In einem zweiten Schritt 11 wird ebenfalls durch die kinematische Kette 2 des robotischen Geräts 1 über die Bohrhülse 5 eine ausgewählte Anpresskraft auf den Knochen des Patienten ausgeübt. Die Richtung der Anpresskraft ist dabei z.B. orthogonal zur Oberfläche des Knochens oder parallel zur geplanten Bohrtrajektorie für die Schraube. Die Anpresskraft kann dabei derart moderat gewählt sein, dass die Bohrhülse bei statischem Knochen den Kontakt zur Oberfläche des Knochens gerade nicht verliert. Bevorzugt wird keine sehr starke Anpresskraft ausgeübt, um den Knochen nicht zu beschädigen.

In einem dritten Schritt 12 wird anschließend durch die Kraftsensoren 6 an der kinematischen Kette eine auf die Bohrhülse einwirkende insbesondere externe Kraft in allen drei Dimensionen gemessen bzw. ein dazu proportionaler Wert gemessen und die einwirkende Kraft bestimmt. Die einwirkende Kraft kann z.B. durch Patientenbewegungen und andere externe Einflüsse hervorgerufen werden. Die Kraftsensoren geben die Informationen über die gemessenen/ bestimmten Kraft an die Steuerungs- und Regelungseinrichtung weiter. Auf der Basis dieser gemessenen Kraft reguliert dann in einem vierten Schritt 13 die Steuerungs- und Regelungseinrichtung des robotischen Geräts mittels Impedanzregelung die Anpresskraft innerhalb eines Toleranzbereichs, also im Rahmen von vorbestimmten Abweichungen um die ausgewählte Anpresskraft. Leichte Bewegungen des Knochens werden dadurch wie bei einem Dämpfungssystem abgefedert und die Bohrhülse verliert nicht den Kontakt zum Knochen. Die Messung und Regelung auf Basis der Messungen können über einen bestimmten Zeitraum, z.B. Dauer des Eingriffs, kontinuierlich durchgeführt werden.

Durch das erfindungsgemäße medizinisch-robotische Gerät wird ein automatisches System zur Stabilisierung der Bohrhülse auf dem Knochen geschaffen. Im Gegensatz zum manuellen Halten der Bohrhülse kann der Chirurg seinen Workflow nachhaltig verbessern. Im Vergleich zu bekannten manuellen oder (halb-) automatischen Verfahren wird die Präzision sowohl in Bezug auf die Position als auch die Orientierung der in den Knochen einzuführenden Schraube verbessert. Es werden auf einfache, effektive und für den Patienten sichere und schonende Weise Patientenbewegungen und andere externe einwirkende Kräfte auf die Bohrhülse abgefedert und es wird verhindert, dass die Bohrhülse verrutscht und dabei Gewebe des Patienten verletzt wird.

Die Erfindung lässt sich in folgender Weise kurz zusammenfassen: Das erfindungsgemäße medizinisch-robotische Gerät weist eine kinematischen Kette von bewegbaren Komponenten, einen am Ende der kinematischen Kette angeordneten Endeffektor in Form einer Bohrhülse, welche Bohrhülse dazu ausgebildet ist, zum Einführen einer Schraube in ein Objekt (z.B. ein Knochen eines Patienten) auf das Objekt aufgelegt zu werden, wobei die Bohrhülse an ihrem auf das Objekt auflegbaren Ende eine strukturierte Oberfläche aufweist, zumindest einen Sensor an der kinematischen Kette zum Erfassen von eine auf die Bohrhülse einwirkende Kraft repräsentierenden Werten und eine Steuerungs- und Regelungseinrichtung zur Steuerung und Regelung der kinematischen Kette auf, wobei die Steuerungs- und Regelungseinrichtung dazu ausgebildet ist, mittels Impedanzregelung unter Berücksichtigung der erfassten Werte eine ausgewählte Anpresskraft der Bohrhülse auf das Objekt innerhalb eines Toleranzbereichs zu regeln.

## Patentansprüche

1. Medizinisch-robotisches Gerät (1) aufweisend
• eine kinematischen Kette (2) von bewegbaren Komponenten (3),
• einen am Ende der kinematischen Kette (2) angeordneten Endeffektor in Form einer Bohrhülse (5), welche Bohrhülse (5) dazu ausgebildet ist, zum Einführen einer Schraube in ein Objekt auf das Objekt aufgelegt zu werden, wobei die Bohrhülse (5) an ihrem auf das Objekt auflegbaren Ende eine strukturierte Oberfläche (7) aufweist,
• zumindest einen Sensor an der kinematischen Kette (2) zum Erfassen von eine auf die Bohrhülse 85) einwirkende Kraft repräsentierenden Werten,
• eine Steuerungs- und Regelungseinrichtung (8) zur Steuerung und Regelung der kinematischen Kette (2),
wobei die Steuerungs- und Regelungseinrichtung (8) dazu ausgebildet ist, mittels Impedanzregelung unter Berücksichtigung der erfassten Werte eine ausgewählte Anpresskraft der Bohrhülse (5) auf das Objekt innerhalb eines Toleranzbereichs zu regeln.

2. Medizinisch-robotisches Gerät nach Anspruch 1, wobei der zumindest eine Sensor als Kraft- oder Drehmomentsensor (6) ausgebildet ist.

3. Medizinisch-robotisches Gerät nach Anspruch 1 oder 2, wobei der zumindest eine Sensor zum Erfassen der eine auf die Bohrhülse (5) in drei Dimensionen einwirkende Kraft repräsentierenden Werte ausgebildet ist.

4. Medizinisch-robotisches Gerät nach einem der vorangehenden Ansprüche, wobei die Bohrhülse (5) an ihrem auf das Objekt auflegbaren Ende eine gezahnte Oberfläche (7) aufweist.

5. Medizinisch-robotisches Gerät nach einem der vorangehenden Ansprüche, wobei die Steuerungs- und Regelungseinrichtung (8) dazu ausgebildet ist, die ausgewählte Anpresskraft der Bohrhülse (5) auf das Objekt entsprechend einem Federungssystem zu regeln.

6. Verfahren zur Regelung einer ausgewählten Anpresskraft einer Bohrhülse (5), welche am Ende einer kinematischen Kette (2) von bewegbaren Komponenten (3) eines medizinisch-robotischen Geräts (1) angeordnet ist und dazu ausgebildet ist, zum Einführen einer Schraube in ein Objekt auf das Objekt aufgelegt zu werden und welche Bohrhülse (5) an ihrem auf das Objekt auflegbaren Ende eine strukturierte Oberfläche (7) aufweist, auf das Objekt durch eine dem medizinisch-robotischen Gerät (1) zugeordneten Steuerungs- und Regelungseinrichtung (8) mit den folgenden Schritten:
• Aufbringen der strukturierten Oberfläche 87) der Bohrhülse (5) auf das Objekt,
• Ausüben einer ausgewählten Anpresskraft der Bohrhülse (5) auf das Objekt durch das medizinisch-robotische Gerät (1),
• Erfassen einer auf die Bohrhülse (1) einwirkenden Kraft, insbesondere in drei Dimensionen, wobei zumindest ein die Kraft repräsentierender Wert mittels zumindest eines an der kinematischen Kette (2) angeordneten Sensors, insbesondere Kraft- oder Drehmomentsensors (6), gemessen wird, und
• Regeln der ausgewählten Anpresskraft der Bohrhülse (5) auf das Objekt innerhalb eines Toleranzbereichs mittels Impedanzregelung unter Berücksichtigung der einwirkenden Kraft.

7. Verfahren nach Anspruch 6, wobei die ausgewählte Anpresskraft der Bohrhülse (5) auf das Objekt entsprechend einem Federungssystem geregelt wird.
